# EUROPEAN PATENT APPLICATION

(11) **EP 1 806 102 A1**
(43) Date of publication of application: **11.07.2007**
(21) Application number: 06026840.6
(22) Date of filing: 22.12.2006
(51) Int. Cl.: A61B 17/064

(54) **Multi-pronged compressive absorbable tack**

(30) Priority: 06.01.2006 US 326926
(71) Applicant: Tyco Healthcare Group LP, North Haven, CT 06473 (US)
(72) Inventor: Aranyi, Ernest, Easton, CT 06612 (US); Zergiebel, Earl M., Guilford, CT 06430 (US); Criscuolo, Christopher J., Branford, CT 06405 (US)
(74) Representative: HOFFMANN EITLE

(57) **Abstract**

A multi-pronged compressive absorbable tack (120) is disclosed which generally includes a backspan (122) and a plurality of prongs (128,130) extending distally from the backspan (122). Each prong (128,130) terminates in a distal tip (128,130) having a base (132,134), with a flat proximal-facing surface (144,146), and a conical portion (136,138) extending distally therefrom. In certain embodiments, one or more intermediate barbs (152,154,170,174) are provided along the length of the prongs (128,130). The barbs (152,154,170,174) include a base as depicted in Fig. 3B having a flat proximal-facing surface (156,158,178,180), and a conical portion (160,162,182,184) extending distally therefrom. A surgical instrument as depicted in Fig. 4 for applying one or more tacks to tissue is also disclosed. A method of applying the disclosed tacks to secure a mesh against tissue is also disclosed.

## Description

### BACKGROUND

The present disclosure relates to compressive tacks for use in surgical procedures. More particularly, the present disclosure relates to compressive tacks, instruments and methods for use in attaching mesh to tissue in hernia repair procedures. Inaccuracies

Surgical tacks or staples are used in a wide variety of surgical procedures to close incisions or openings in tissue, as well as to secure various prosthetics, such as, for example, mesh to tissue. Many known surgical tacks or staples are similar to conventional staples having relatively smooth straight legs for being driven into tissue. When these type of tacks or staples are used they provide little or no anchoring in tissue to prevent the tack or staple from working out of, or being pulled out of, the tissue. Additionally, these type of surgical tacks or staples, when used on opposed sides of an incision or hernial opening, do not compress the tissues together. Thus, the fusion or healing of the adjacent tissues together may not be complete or optimal.

Thus, it may be desirable to provide a surgical tack having structures that would hinder inadvertent pullout of the tack from tissue. It may also be desirable to provide a surgical tack having structure that would inwardly compress tissue on opposed sides of an incision to facilitate complete healing of adjacent tissue. Additionally, it may be desirable to have a surgical instrument capable of applying surgical tacks having these anchoring and tissue compression structures.

### SUMMARY

There are disclosed compressive, absorbable tacks for use in surgical procedures, such as hernia repair procedures, to secure a mesh to tissue. One embodiment of the disclosed tack generally includes a backspan and a pair of prongs extending distally from the backspan. Each of the prongs terminates in a tip having a base. The pair of prongs each have a first diameter and the base of each tip has a second diameter greater than the first diameter. Each tip further has a conical portion extending distally from the base. A distal end of each of the conical portions can form a blunt or a sharp point. In the disclosed embodiments the tack is formed of an absorbable material.

At least a portion of the bases of the tips have relatively flat proximal-facing surfaces. In the embodiments disclosed herein, the flat proximal-facing surfaces are oriented substantially perpendicular to a longitudinal axis of the respective prongs.

The backspan has a width and a length between the prongs, the width of the backspan is greater than the first diameter of the prong. Additionally, in certain embodiments, the backspan has at least one notch for receipt of a driving mechanism of a surgical instrument.

In an embodiment, the tack includes a backspan with three prongs extending distally therefrom.

In certain embodiments, each prong has at least one barb intermediate the backspan and the tip. Each barb has a base having a third diameter greater than the first diameter of the prong. In particular embodiments, the third diameter is substantially equal to the second diameter.

Each barb has a conical portion which extends distally from the respective base. The conical portion of the barb extends partially around the prong. The conical portion of the barb is formed on an inner surface of the prong to form an inwardly directed tissue compression surface.

The base of each tip has a substantially flat proximal-facing surface. The flat proximal-facing surface of the base is generally oriented perpendicular to the longitudinal axis of the prong.

In a particular embodiment, the tack includes at least two barbs formed intermediate the backspan and the tip of the prong.

There is also disclosed a compression tack for use in a hernia repair surgery which includes a backspan, a plurality of prongs extending distally from the backspan, a tip formed on a distal end of each of the prongs, each tip having a base and a conical portion extending distally from the base, and at least one barb formed on an inner surface of the prong, each barb having an inwardly directed conical tissue-compressing surface.

A method of attaching a hernia mesh to tissue is also disclosed which includes providing a surgical tack having a backspan and a plurality of prongs extending distally from the backspan, each prong terminating in a tip having a base, wherein the plurality of prongs each has a first diameter and the base of each tip has a second diameter greater than the first diameter. A hernia mesh is placed over tissue and at least one tack is driven through the mesh and into tissue. At least a first area of tissue is compressed between the tips of the tack. Additionally, at least a second area of tissue can be compressed between the prongs by providing at least one inwardly-directed barb intermediate the backspan and the tip.

### DESCRIPTION OF THE DRAWINGS

Various embodiments of the presently disclosed tacks are disclosed herein with reference to the drawings, wherein:

FIG. 1 A is a perspective view of a two-pronged compressive tack;

FIG. 1B is a side view of the tack of FIG. 1A;

FIG. 1C is an alternate side view of the tack of FIG. 1A;

FIG. 1D is a plan view of the tack of FIG. 1A;

FIG. 2A is a perspective view of a second embodiment of a two-pronged compressive tack having an intermediate barb;

FIG. 2B is a side view of the tack of FIG. 2A;

FIG. 2C is an alternate side view of the tack of FIG. 2A;

FIG. 2D is a plan view of the tack of FIG. 2A;

FIG. 3A is a perspective view of a third embodiment of a two-pronged compressive tack having two intermediate barbs;

FIG. 3B is a side view of the tack of FIG. 3A;

FIG. 3C is an alternate side view of the tack of FIG. 3A;

FIG. 3D is a plan view of the tack of FIG. 3A;

FIG. 4 is a perspective view of a tack-applying surgical instrument;

FIG. 5 is a perspective view of a distal end of the tack-applying surgical instrument illustrating a driving mechanism;

FIG. 6 is a perspective view of the distal end of the tack-applying instrument;

FIG. 7 is a side view, partially shown in section, of the tack securing a mesh to tissue; and

FIG. 8 is a perspective view of a three-pronged compressive tack.

### DETAILED DESCRIPTION

Embodiments of the presently disclosed tack will now be described in detail with reference to the drawings wherein like numerals designate identical or corresponding elements in each of the several views. As is common in the art, the term 'proximal" refers to that part or component closer to the user or operator, e.g., surgeon or physician, while the term "distal" refers to that part or component farther away from the user.

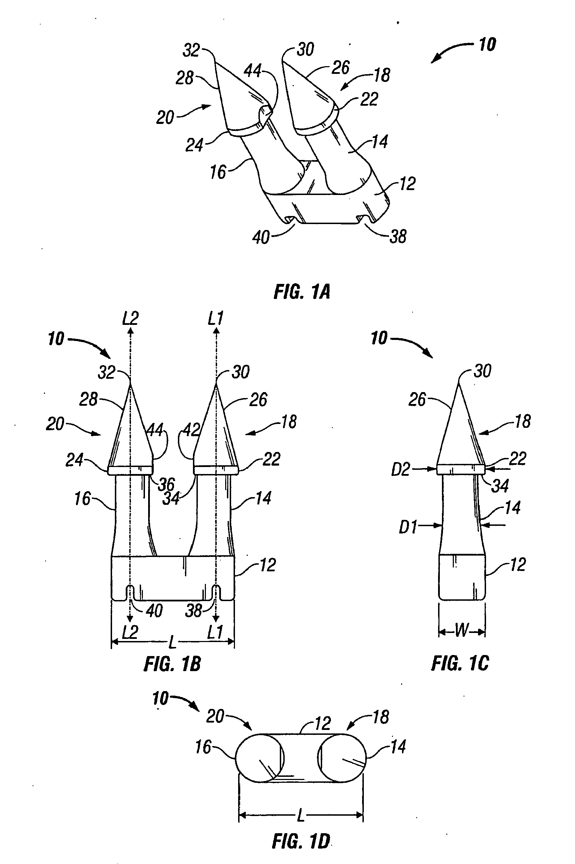

Referring to FIGS. 1A-1D, and initially to FIGS. 1A and 1B, a first embodiment of the presently disclosed multi-pronged compressive tack is disclosed. The tack 10 in this embodiment generally includes a backspan 12 having first and second prongs, 14 and 16, extending distally therefrom. Prongs 14 and 16 terminate in tips 18 and 20, respectively. Tack 10 can be formed of any suitable biocompatible material and, in the specific embodiments disclosed herein, is formed of an absorbable and resorbable material.

Tips 18 and 20 each include bases 22 and 24, respectively, and conical portions 26 and 28 extending distally from bases 22 and 24, respectfully. Conical portions 26 and 28 include distal ends 30 and 32, respectively. Distal ends 30 and 32 may be either blunt or terminate in a sharp point. Base 22 includes a proximal-facing surface 34 configured to facilitate retaining tack 10 within tissue. Similarly, base 24 includes a proximal-facing surface 36. Proximal-facing surfaces 34 and 36 are oriented substantially perpendicular to longitudinal axes L1 and L2 of prongs 14 and 16, respectively (shown in FIG. 1B).

As best shown in FIG. 1C, prongs 14 and 16 generally have a first diameter D1 and bases 22 and 24 of tips 18 and 20 have a second diameter D2 greater than first diameter D1. Conical portions 26 and 28, extend distally from bases 22 and 24, respectfully, and form inwardly directed compressive surfaces on tips 18 and 22 which compress tissue as tack 10 is inserted therethrough. By compressing tissue, such as tissue adjacent a hernia opening or an incision, between conical portions 26 and 28 of tips 18 and 22 healing of the tissue therebetween is enhanced. Additionally, the larger amount of tissue that is placed between the tips 18 and 22, the tighter the tack 10 compresses the tissue. A large amount of tissue between the tips 18 and 22 also helps maintain the tack 10 in place.

Referring to FIGS. 1B-1D, backspan 12 has a width W and a length L. Width W is larger than first diameter D1 to allow backspan 12 to secure the surgical mesh against tissue.

Referring for the moment to FIGS. 1A and 1B, backspan 12 of tack 10 may also include notches 38 and 40. Notches 38 and 40 facilitate the engagement with a driving mechanism of a tack applying instrument. Additionally, tips 18 and 20 of tack 10 may include cut outs or second notches 42 and 44 formed in bases 22 and 24 and/or conical portions 26 and 28. Second notches 42 and 44 increase the gap between the two prongs 14 and 16 so that a larger amount of tissue can fit therebetween.

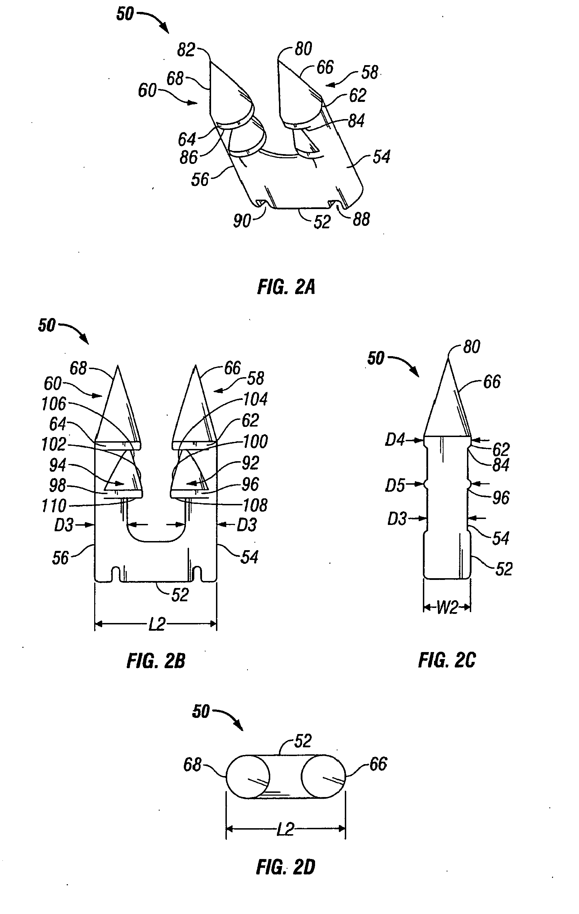

Referring to FIGS. 2A-2D, and initially to FIGS. 2A and 2B, a second embodiment of a compressive tack is disclosed. As with tack 10, tack 50 includes a backspan 52 and a pair of prongs 54 and 56 extending distally from backspan 52. Prongs 54 and 56 terminate in tips 58 and 60. Tips 58 and 60 each include respective bases 62 and 64 and conical portions 66 and 68 extending distally from bases 62 and 64. Distal ends 80 and 82 of conical portions 66 and 68 can be formed with either blunt or sharp tips as in the proceeding embodiment.

Bases 62 and 64 also have substantially flat proximal-facing surfaces 84 and 86, respectively, to facilitate securing tack 50 within tissue. A pair of notches 88 and 90 are formed in backspan to facilitate advancement through a tack applying surgical instrument.

As best shown in FIG. 2B and 2C, prongs 54 and 56 have a diameter D3. Bases 62 and 64 have a diameter D4 which is greater than diameter D3 of prong 54. Referring for the moment to FIGS. 2C and 2D, backspan 52 has a length L2 and a width W2. Width W2 is also greater than diameter D3.

Tack 50 is further provided with a pair of protrusions or barbs 92 and 94 intermediate backspan 52 and prongs 54 and 56. Barbs 92 and 94 are provided to further compress tissue as tack 50 is inserted into tissue about opposed sides of a hernial opening or incision in tissue. Barbs 92 and 94 include bases 96 and 98, respectively, as well as distally extending conical portions 100,102, respectively.

As best shown in FIG. 2B, distal tips 104, 106 of conical portions 100,102 extend distally towards bases 62 and 64 of tips 58 and 60. This allows for a continued compression of tissue as tack 50 is inserted into tissue. Additionally, bases 96 and 98 include respective flat proximal-facing surfaces 108 and 110 to facilitate securing tack 50 within tissue.

Referring again to FIGS. 2B and 2C, bases 96 and 98 have diameter D5 which are greater than diameter D3 of prongs 54 and 56.

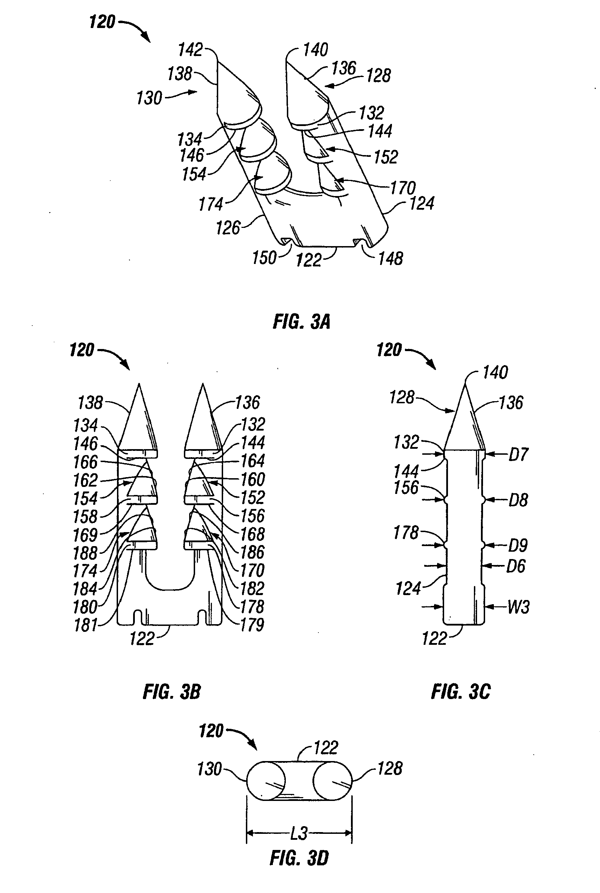

Referring now to FIGS. 3A to 3D, and initially with reference to FIGS. 3A and 3B, a third embodiment of a two-pronged compressive tack is disclosed. As with previously disclosed tacks, tack 120 includes a backspan 122 and a pair of prongs 124 at 126 extending distally from backspan 122. Tips 128 and 130 are formed on the distal ends of prongs 124 and 126, respectively. Tips 128 and 130 include respective bases 132 and 134 and conical portions 136 and 138 extending distally from bases 132 and 134. Conical portions 136, 138 include distal ends 140 on 142, respectively, which may be either blunt or sharpened. Bases 132 and 134 of tips 128 and 130 include proximal-facing surfaces 144 and 146 to facilitate retaining tack 10 within tissue. Additionally, as with prior embodiments, tack 120 may include a pair of notches 148, 150 formed in backspan 122.

A pair of barbs, such as first barbs 152 and 154, are formed along inner surfaces of prongs 124 and 126. Barbs 152 and 154 include respective bases 156 and 168 as well as conical portions 160 and 162 extending distally from bases 156 and 158. Distal ends 164, 166 of conical portions 160, 162 extend distally to bases 132, 134 of tips 128 and 130. Bases 156 and 158 of barbs 152 and 154 also include relatively flat proximal-facing surfaces 168 and 169 thereby further facilitate securing tack 120 within tissue. As with prior embodiments, conical portions 160 and 162 form compressive surfaces to force opposed edges of a hernial opening, or an incision in tissue, together to facilitate healing after the tack is installed.

In this particular embodiment, a second pair of barbs, such as, for example, barbs 170 and 174 are provided intermediate barbs 152 and 154 and backspan 122. This second pair of barbs 170, 174 further facilitates compression of tissue within the tack 120 and retention of the tack 120 within tissue. As shown, second pair of barbs 170, 174 include respective bases 178 and 180. Bases 178 and 180 include relatively flat proximal-facing surfaces 179 and 181, respectively. Conical portions 182, 184 extend distally from bases 178 and 180 such that distal ends 186, 188 of conical portions 182 and 184 extend distally towards bases 156, 158 of first pair of barbs 152, 154.

Similar to previous embodiments, prongs 124 and 126 have a diameter D6 which is smaller than the diameter D7 of bases 132, 134 of tips 128, 130. Additionally, the diameter D8 of bases 156 and 158 of first barbs 152 and 154 is greater than the diameter D6 of prongs 124 and 126. A diameter D7 of bases 132 and 134 is substantially equal to a diameter D8 of bases 156 and 158. Further, bases 178 and 180 of second set of barbs 172 and 174 have a diameter of D9 which is greater than the diameter D6 of prongs 124 and 126 and may be substantially equal to D7 and/or D8. Additionally, backspan 122 has a length L3 and a width W3. Width W3 is greater than diameter D6 of prongs 124 and 126 and may be substantially equal to any or all of the diameters D7, D8 or D9. As noted above, a relative wider diameter D3 of backspan 122 helps secure tack 120 against mesh applied to tissue.

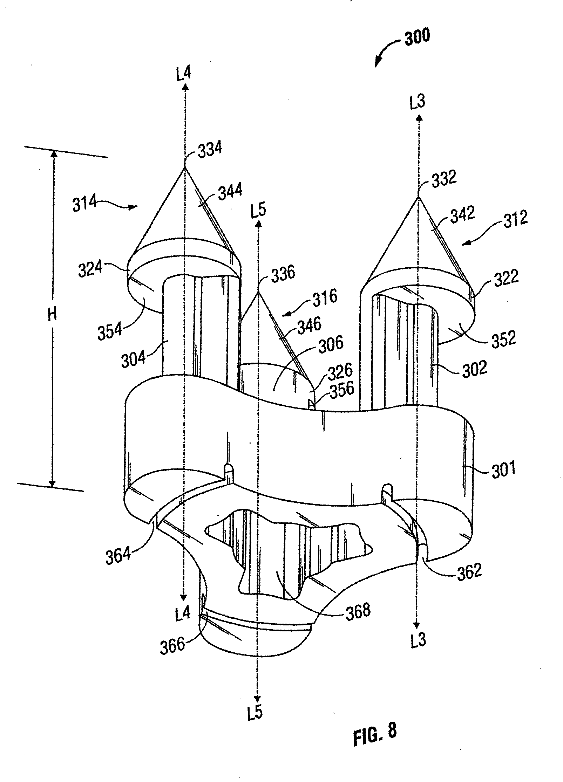

With reference to FIG. 8, a three-pronged tack 300 is illustrated. The tack 300 comprises three prongs 302, 304, 306 extending distally from a backspan 301. The prongs 302, 304, 306 terminate in tips 312, 314, 316, respectively. The tips 312, 314, 316 each include a base 322, 324, 326, respectively, and conical portions 342, 344, 346 extending distally from the bases 322, 324, 326, respectively. The conical portions 342, 344, 346 include distal ends 332, 334, 336, respectively. Distal ends 332, 334, 336 may be either blunt or terminate in a sharp point. The bases 322, 324, 326 includes proximal-facing surfaces 352, 254, 356, respectively, which are substantially perpendicular to the axes L3, L4, L5 through the prongs 312, 314, 316, respectively. The midpoint of a the backspan 301 also defines a longitudinal axis (not shown for clarity), defined hereinafter as the midpoint axis.

It is envisioned for the three-pronged tack 300 to include a plurality of barbs disposed thereon, similar to the barbs described and illustrated with reference to FIGS. 2A - 2D and FIGS. 3A - 3D.

As illustrated in FIG. 8, vis-à-vis FIGS. 1A - 1D and FIGS. 2A - 2D, the tips 312, 314, 316 extend outwardly from their respective prongs 302, 304, 306 (i.e., away from the midpoint axis), such that the proximal-facing surfaces 352, 354, 356 are larger on the outward side (i.e., away from the midpoint axis) than on the inward side (i.e., towards the midpoint axis). In contrast, in FIGS. 2A- 2D, the tips 58, 60 extend inwardly. In further contrast, in FIGS. 1A-1D, the tips 18, 20 are substantially centered with respect to their respective prongs 14, 16. It is envisioned for any of the embodiments to comprise either type of tip (i.e., extending outwardly, extending inwardly, centered), or any combinations thereof.

The height H of the tack 300 is illustrated in FIG. 8. The height H of the tack 300 may be in the range of about 3.0 mm to about 6.0 mm. More specifically, the height H of the tack 300 may be in the range of about 3.0 mm to about 6.0 mm. Similarly, the height of the tacks in the other embodiments may be of similar distances.

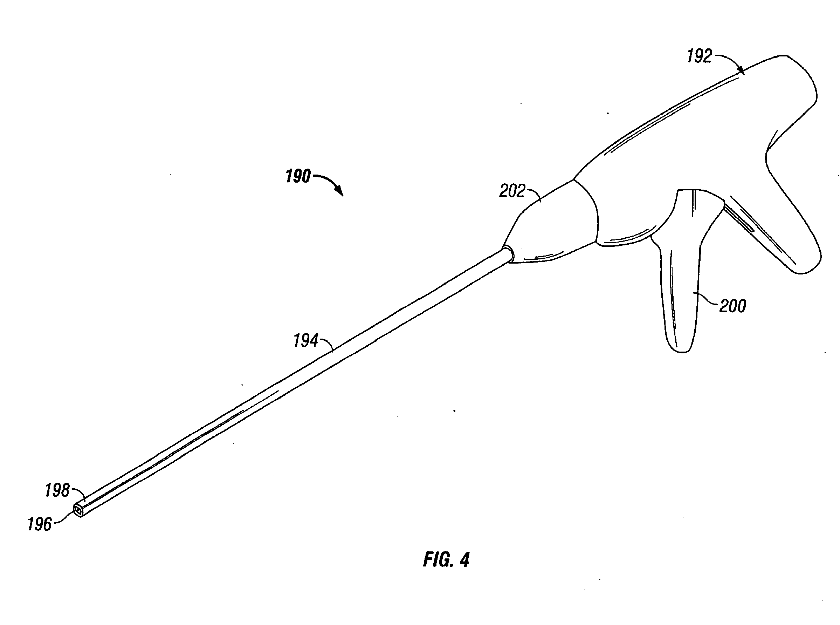

In an exemplary embodiment, the backspan 301 comprises three notches 362, 364, 366 therein for engagement with a surgical instrument (similar to the surgical instrument 190 depicted in FIGS. 4-6, discussed hereinbelow). It is envisioned for the backspan 301 to comprise a bore 368 therethrough. Other features of the tack 300 may be similar to the features of the tacks described with reference to other embodiments herein.

The presently disclosed tacks are suitable for use in a variety of surgical procedures. The disclosed tacks are particularly suitable for use in a hernia repair procedure where the tack is used to secure a hernia mesh over a hernial opening. Referring now to FIG. 4, there is disclosed a surgical tack-applying instrument particularly suited for use with the previously disclosed tacks of FIGS. 1-3D. A similar instrument can be used with the tack 300 having three prongs 302, 304, 306, as shown in FIG. 8, as can be appreciated. Instrument 190 generally includes a pistol grip handle 192 having an elongated tubular member 194 extending distally from pistol grip handle 192. A plurality of the previously disclosed tacks are contained within a magazine within elongated tubular member 194 and are ejectable from surgical instrument 190 through an opening 196 in a distal end 198 of elongated tubular member 194. A trigger 200 is provided on pistol grip handle 192 to actuate instrument 190. Additionally, instrument 190 may be provided with a rotation collar 202 to rotate elongated tubular member 194 and thus vary the orientation of distal opening 196 for appropriate tissue orientation.

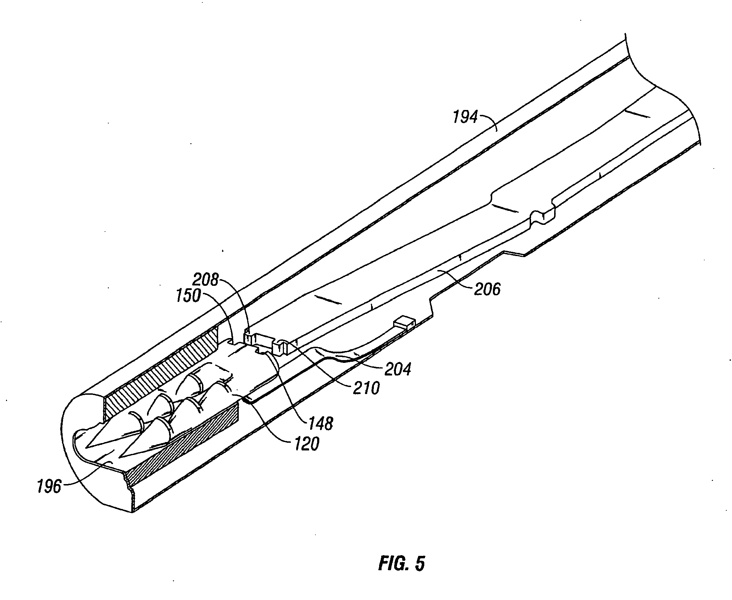

Referring to FIG. 5, the distal end 198 of the elongated tubular member 194 is shown. As discussed above, a tack, such as, for example, tack 120, is contained within a magazine (not shown) within elongated tubular member 194 and may be ejected from distal opening 196 upon actuation of the trigger 200 of surgical instrument 190. Successive tacks 120 are biased towards the distal opening 196 by a leaf spring 204. Once positioned within distal opening 196, tacks 120 can be driven out of instrument 190 upon actuation thereof by means of a driving mechanism 206. As shown, driving mechanism 206 is provided with a pair of projections 208, 210 formed on a distal end thereof. Projections 208, 210 are configured to engage notches 148, 150 in tack 120 to facilitate proper alignment of tack 120 as it is driven out of instrument 190. In this manner, upon actuation of trigger 200, successive series of tacks 120 can be ejected out of distal opening 196 and applied to tissue as desired.

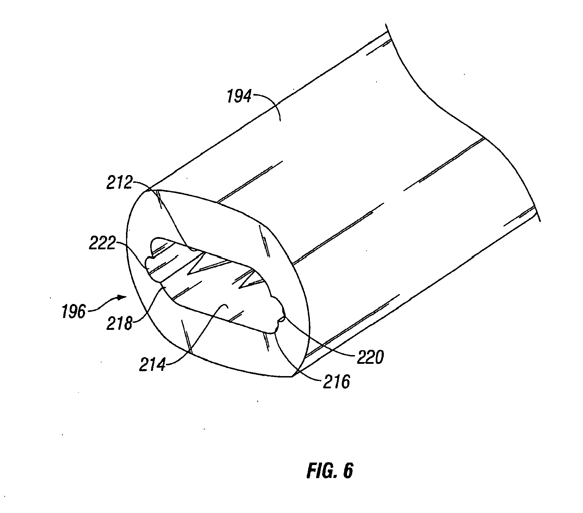

Referring for the moment to FIG. 6, an enlarged perspective view of the distal end 198 of the elongated tubular member 194 including distal opening 196 is shown. Distal opening 196 is generally rectangular and has upper and lower surfaces 212, 214, respectively, connected by side surfaces 216, 218. Cut outs or notches and 222 are formed in side surfaces 216, 218 to accommodate the rounded diameter of prongs 124, 126 of tack 120 as well as the diameters of bases 132 and 134 of tips 128 and 130, bases 156, 158 of first barbs 152 and 154, and bases 178 and 180 of second set of barbs 172 and 174. The notches 220 and 222 in side surfaces 216 and 218 may prevent twisting of tack 120 upon ejection from surgical instrument 190.

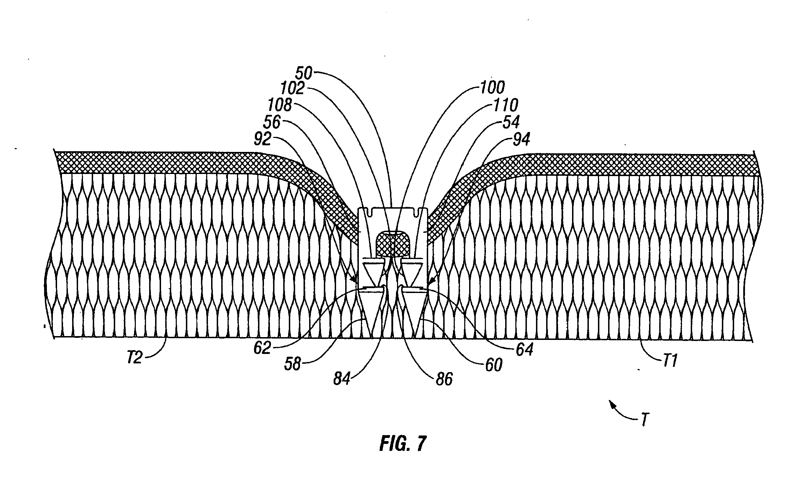

The use of one of the previously disclosed tacks such as, for example, tack 50 to secure a mesh about a hernial opening will now be described. Referring to FIG. 7, and as noted above, during a hernia repair procedure the hernial site is accessed generally laparoscopically and a surgical instrument introduced into the body. A piece of surgical mesh such as, for example, surgical mesh M is positioned over the defect or hernial opening.

In use, the presently disclosed surgical tacks, such as, for example, tack 50 is fired or ejected from surgical instrument 190 through mesh M and into tissue T such that prong 54 engages and is inserted into a first tissue section T1 on a first side of the hernial opening and prong 56 engages and is inserted into a second tissue section T2 on an opposed side of the hernial opening (see FIG. 7). As discussed hereinabove, flat proximal-facing surfaces 84, 86 of bases 62, 64 of tips 58, 60 assist retaining tack 50 within tissue T. When the tack 50 used in the hernia repair procedure includes intermediate barbs, such as barbs 92 and 94 of tack 50, proximal-facing surfaces 108, 110 of bases 96 and 98 of barbs 92 and 94 further facilitate retaining tack 50 within tissue T.

Additionally, the inwardly-directed faces of conical portions 66 and 68 of tips 58 and 60 serve to compress tissue as tack 50 is inserted into the tissues. Further, the inwardly-directed conical portions 100 and 102 of barbs 92 and 94 further serve to compress tissue when tack 50 is inserted therein.

It is envisioned for at least a portion of the tacks 10, 50, 120, 300 to be made from polymers. Bio-absorbable materials used for the tacks 10, 50, 120, 300 may include any bioresorbable polymer or copolymer known to those skilled in the art, so long as the polymer utilized has sufficient strength and possesses the necessary mechanical properties to permit formation. Suitable polymers which may be utilized to form the tacks include, but are not limited to, trimethylene carbonate, caprolactone, diozanone, glycolic acid, lactic acid, glycolide, lactide, homopolymers thereof, copolymers thereof, and combinations thereof.

It will be understood that various modifications may be made to the embodiments disclosed herein. For example, more than two barbs may be provided along the length of the prongs. Further, the number of barbs provided on each prong may be different. Additionally, the bases and conical portions of the barbs may be completely circumferential about the prong. Still further, the proximal-facing the surfaces of the barbs and tips may be oriented at an angle to the prong and may assume surface shapes other than flat, such as, for example, conical, concave, etc. Therefore, the above description should not be construed as limiting, but merely as exemplifications of particular embodiments. Those skilled in the art will envision other modifications within the scope and spirit of the claims appended hereto.

## Claims

1. A tack for use in surgical procedures comprising:
a backspan;
a plurality of prongs extending distally from the backspan, each prong terminating in a tip having a base, wherein each of the plurality of prongs has a first diameter and the base of each tip has a second diameter greater than the first diameter.

2. The tack as claimed in claim 1, wherein each tip comprises a conical portion extending distally from the base.

3. The tack as claimed in claim 2, wherein a distal end of at least one conical portion forms a blunt point.

4. The tack as claimed in any one of the preceding claims, wherein at least a portion of the tack is formed of an absorbable and resorbable material.

5. The tack as claimed in any one of the preceding claims, wherein at least a portion of at least one base comprises a relatively flat proximal-facing surface.

6. The tack as claimed in claim 5, wherein the flat proximal-facing surface is oriented substantially perpendicular to a longitudinal axis of at least one prong.

7. The tack as claimed in any one of the preceding claims, wherein the backspan comprises a width and a length between the prongs, the width of the backspan being greater than the first diameter of at least one prong.

8. The tack as claimed in any one of the preceding claims, wherein the backspan comprises at least one notch disposed on a proximal portion thereof.

9. The tack as claimed in any one of the preceding claims, wherein each prong comprises at least one barb intermediate the backspan and the tip.

10. The tack as claimed in claim 9, wherein each barb comprises a base having a third diameter greater than the first diameter of at least one prong.

11. The tack as claimed in claim 10, wherein the third diameter is substantially equal to the second diameter.

12. The tack as claimed in claim 9, 10 or 11, wherein each barb comprises a distally-extending conical portion.

13. The tack as claimed in claim 12, wherein the conical portion of each barb extends partially around the prong.

14. The tack as claimed in claim 12 or 13, wherein the conical portion of each barb is formed on an inner surface of the prong to form an inwardly-directed compression surface.

15. The tack as claimed in any one of claims 9 to 14, wherein the base of at least one barb comprises a substantially flat proximal-facing surface.

16. The tack as claimed in claim 15, wherein the substantially flat proximal-facing surface of the base is substantially perpendicular to a longitudinal axis of at least one prong.

17. The tack as claimed in any one of claims 9 to 16, further comprising at least two barbs formed intermediate the backspan and the tip of at least one prong.

18. The tack as claimed in any one of the preceding claims, wherein the tack comprises two prongs.

19. The tack as claimed in any one of the preceding claims, wherein the tack comprises three prongs.

20. The tack as claimed in any one of the preceding claims, wherein the distance from a proximal portion of the backspan to a distal portion of a tip is in the range of from 3.0 mm to 6.0 mm.

21. A compression tack for use in a hernia repair surgery comprising:
a backspan;
a plurality of prongs extending distally form the backspan;
a tip formed on a distal end of each of the plurality of prongs, each tip comprising a base and a conical portion extending distally from the base; and
at least one barb formed on an inner surface of the plurality of prongs, each barb comprising an inwardly-directed conical tissue compressing surface.

22. A method of attaching mesh to tissue comprising the steps of:
providing a surgical tack having a backspan and a plurality of prongs extending distally from the backspan, each of the plurality of prongs terminating in a tip comprising a base, wherein each of the plurality of prongs comprises a first diameter and the base of each tip has a second diameter greater than the first diameter;
placing a mesh over tissue;
driving the surgical tack through the mesh and into tissue; and
compressing at least a first area of tissue between at least two of the plurality of prongs.

23. The method according to claim 22, further comprising the step of compressing at least a second area of tissue between at least two of the plurality of prongs by providing at least one inwardly directed barb intermediate the backspan and the tip.

24. An instrument for applying surgical tacks, comprising:
a handle;
an elongate member extending distally from the handle and comprising a distal end, the distal end comprising a distal opening, the elongate member configured to house a plurality of tacks;
a trigger disposed on the handle; and
a driving mechanism comprising a plurality of projections formed on a distal end thereof, the projections configured to engage notches on a tack,
whereby, upon actuation of the trigger, a tack may be ejected from the distal opening of the elongate member towards tissue.

25. The instrument as claimed in claim 24, further comprising a rotation collar which is capable of rotating the elongate member.

26. The instrument as claimed in claim 24 or 25, further comprising a leaf spring which biases at last one tack towards the distal opening of the elongate member.
